# EUROPEAN PATENT SPECIFICATION

(11) **EP 3 687 386 B1**
(45) Date of publication and mention of the grant of the patent: **19.07.2023**
(21) Application number: 18815384.5
(22) Date of filing: 01.11.2018
(51) Int. Cl.: A61B 5/021, A61B 5/0235, A61B 5/022, A61B 5/00, A61B 5/0225

(54) **METHOD FOR USE IN AN OPTIMIZATION OF A NON-INVASIVE BLOOD PRESSURE MEASUREMENT DEVICE**
VERFAHREN ZUR VERWENDUNG IN DER OPTIMIERUNG EINER NICHTINVASIVEN BLUTDRUCKMESSVORRICHTUNG
PROCÉDÉ DESTINÉ À ÊTRE UTILISÉ DANS UNE OPTIMISATION D'UN DISPOSITIF DE MESURE DE PRESSION ARTÉRIELLE NON INVASIVE

(30) Priority: 06.11.2017 US 201762582160 P; 30.10.2018 US 201816175661
(43) Date of publication of application: 05.08.2020
(73) Proprietor: Edwards Lifesciences Corporation, Irvine, CA 92614 (US)
(72) Inventor: SCHRAA, Olaf, 1011 GD Amsterdam (NL); VAN GOUDOEVER, Jeroen, Irvine, CA 92614 (US)
(74) Representative: Smith, Jeremy Robert
(86) International application number: PCT/US2018/058715
(87) International publication number: WO 2019/089930

(56) References cited:
- US-A- 4 510 940
- US-A1- 2006 195 034
- US-A1- 2013 023 777

## Description

### TECHNICAL FIELD

The present invention relates to a method for determining whether a match between the configuration of a servo controller of a non-invasive blood pressure measurement device and characteristics of a body part being measured is optimal or sub-optimal.

### BACKGROUND OF THE INVENTION

It has been known for several years how to measure non-invasive blood pressure waveform continuously wherein a pressure cuff is placed around a body extremity, such as a finger. EP 0 048 060 for instance describes that the pressure of a fluid inside the cuff is controlled on the basis of a signal of a plethysmograph by a pressure valve, in turn controlled by a servo control feedback loop.

The signal of the plethysmograph is representing the volume of blood inside the blood vessels of the finger under the cuff. The more blood, the more light from a light source of the plethysmograph is absorbed, which results in a lower signal of the plethysmograph (and vice versa). During every heartbeat, blood is transported through the blood vessels in the finger. This causes a blood pressure and volume increase of the vessels, and thus a signal decrease of the plethysmograph.

In the known method, the cuff pressure of the pressure cuff is servo controlled using a valve, such that the signal of the plethysmograph, and thus the volume of blood inside the blood vessels under the cuff, is kept constant. The pressure exerted on the blood vessel walls from the inside by the heart pulsations is continuously counteracted by a pressure exerted by the pressure cuff, which results in a constant diameter of the blood vessels and, if the set point of the servo is chosen correctly, in an unloading of the vessels. In this case, the counter pressure exerted by the cuff is a direct measure for the actual blood pressure inside the blood vessel, and allows for a continuous non-invasive blood pressure measurement. Ideally, the speed in which the counter pressure exerted by the pressure cuff changes matches the speed in which the blood pressure inside the blood vessels under the cuff changes.

US 2013/023777 A1 discloses a method in which blood pressure is measured using a non-invasive device, and the measured blood pressure is corrected for measurement error caused by oscillations in the response of the cuff pressure control system. In this document, servo gain is adjusted in order to ensure that the control deviation is less than or equal to a prescribed value.

It is therefore an objective of the present invention to provide an improved method for use in an optimization of a non-invasive blood pressure measurement device.

### SUMMARY

The presently claimed invention is defined by appended claims 1 to 13. In a first aspect, the presently claimed invention provides a method for determining whether a match between the configuration of a servo controller of a non-invasive blood pressure measurement device and characteristics of a body part being measured is optimal or sub-optimal, comprising the steps of: **a)** measuring a blood pressure related parameter with a non-invasive blood pressure measurement device, which device comprises at least one servo controller in a first configuration and an inflatable pressure cuff, wherein inflation of the pressure cuff is controlled by the servo controller; **b)** changing the settings, in particular the gain, of the servo controller; **c)** monitoring the change of the blood pressure related parameter under the influence of the changing settings of the servo controller; and **d)** determining whether a match between the servo controller configuration and characteristics of the body part being measured is optimal or sub-optimal, based on the monitoring of the change of the blood pressure related parameter.

Also described is a method for use in an optimization of a non-invasive blood pressure measurement device, comprising the steps of **a)** measuring a blood pressure related parameter with a non-invasive blood pressure measurement device, which device comprises at least one servo controller in a first configuration; **b)** changing the settings, in particular the gain, of the servo controller; **c)** monitoring the change of the blood pressure related parameter under the influence of the changing settings of the servo controller; and **d)** determining a match between the servo controller configuration and the body part being measured based on the monitoring of the change of the blood pressure related parameter. The match may be optimal, such that the controller configuration and the body part are matched, or the match may be sub-optimal or off, such that a mismatch is determined. The controller may for instance be a PID controller.

It has been found that the servo controls can be optimized for each measured body part, in particular each body extremity, such as the fingers. For example the optimal servo control differs for warm and cold body parts, or fingers. In a cold finger for example the smooth muscles around the arteries inside the finger are contracted more compared to in warm fingers, which contraction limits blood flow to the fingers. This contracted state firms up the fingers, wherein the arteries change or expand relatively slow as well, such that for slow responding arteries an optimised servo system should be used to match this behaviour. The same applies vice versa, for warmer fingers and rapid responses.

One of the parameters in the servo control is the gain. Gain is a proportional value that shows the relationship between the magnitude of the input and the magnitude of the output signal at steady state. By altering the gain one can provide more or less "power" to the system. However, increasing gain or decreasing gain beyond a particular safety zone can cause the system to become unstable, since an increase in signal also increases error margins on the signals.

It has been found that variations in the gain in the servo control have little effect on the servo control when the servo control is matched to the body part that is measured, whereas variations in the gain have large effects on the servo control when the control is not matched. When the gain is changed during measurements, for instance during continuous non-invasive blood pressure measurements, and the change in gain does not particularly affect the results, it may be determined that the servo control is tuned, or matched, to the measured body part. On the other hand, if the change in gain causes a change in the measurement, it may be determined that the servo control is not tuned to the measured body part, and could be improved. Matching in the context of the invention means that the control is optimized for the specific body part characteristics, and that the servo controller is at the right configuration.

The blood pressure related parameter may be chosen from the group of pulse pressure, blood pressure or derivatives thereof. Derivatives for instance include changes in pressures over time. The group may further include parameters like controller errors, indicative of the performance of the control, which in turn are based on blood pressure related parameters or combinations of the parameters.

The method may further comprise the step of **e)** adjusting the configuration of the servo controller in relation to the determination at step **d).** This step is typically taken if the determination of the match, in step **d),** indicated that there is no (complete) match between the controls of the system and the body parts to be measured. By adjusting the configuration of the servo control, the match between the settings and the characteristics of the body part may be improved. One could for instance adapt the frequencies used or modify other servo controller characteristics or parameters to match the body part characteristics.

During step **b)** the settings may be changed for at least one heartbeat, in particular about 2 heartbeats. In order to be able to determine the effect of the change on the blood pressure related parameter, at least one single heartbeat should pass in the changed configuration. Since the blood pressure is variable by nature, the influence of changed servo settings can only be determined statistically, by for example measure one beat with current settings, measure the next beat with some changed servo settings, then again one beat with current settings and repeat this alternation for some period to accumulate sufficient data to determine the influence of the changed servo settings.

During step **b),** the settings may be changed to a value above the original setting as well as to a value below the original setting. This way, the change in blood pressure related parameters may be seen along both ways of the original setting, which could indicated whether or not an increase or a decrease of the original setting could result in an improved configuration, or a configuration which results in a better match with the characteristics of the body part which is measured.

Step **d)** may comprise the steps of calculating the optimal configuration of the servo controller based on the change in settings and resulting change of the blood pressure related parameter and comparing the optimal configuration with the current configuration of the servo controller. When the optimal configuration is calculated from the change in settings, an empirically determination of the best settings can be avoided, which saves time. The method according to the invention may thus include the steps of: **f)** calculating the optimal configuration of the servo controller based on the change in settings and resulting change of the blood pressure related parameter; and **g)** adjusting the configuration of the servo control to the calculated optimal configuration.

Steps **b)** and **c)** may be repeated until the change in settings, in particular the gain, does not result in a change in the blood pressure related parameter, wherein in step **d)** an optimal match is established. When the change in the settings of the servo control does not influence or change the measured parameter, the servo configuration match the characteristics of the measured body part.

The steps may be repeated during measurement with the non-invasive blood pressure measurement device, in particular periodically. This enables a constant monitoring and adjustment of the match between the servo control configuration and the measured body part. For instance, when the muscle contraction in a measured finger changes during the measurement, the periodic check of the match between the finger and the servo settings will indicate that that the characteristics of the finger have changed, and the servo control may be changed because of this.

The settings, in particular the gain, of the servo controller may be normalized before changing. The normalizing may be used to consider the settings to be changed in a safe area, for instance an area which is stable. In particular when the gain is increased, high gains (so high amplifications of the signals) results in oscillation of the signal. In the normalized settings, the highest gain which is stable, and does not result in oscillation, is set to value 1. This normalized gain may for instance be used to determine a starting point for the measurements. One could for instance start the measurement at a normalized gain between 0.4 and 0.6. In turn, the change in the settings of the servo controller may be expressed in normalized gain as well, for instance by increasing or decreasing the gain by 0.1 in normalized gain.

In a second aspect, the presently claimed invention provides a non-invasive blood pressure measurement device comprising a processor configured to execute a method according to the invention, and further comprising at least one servo controller, an inflatable pressure cuff, wherein inflation of the pressure cuff is controlled by the servo controller, and at least one blood pressure measurement tool.

Also described is a non-invasive blood pressure measurement device configured for use in a method according to the invention, comprising at least one servo controller; and at least one blood pressure measurement tool.

The non-invasive blood pressure measurement device may comprise at least two blood pressure measurement tools, wherein a first blood pressure measurement tool may be configured for optimization of a non-invasive blood pressure measurement device, and wherein a second blood pressure measurement tool may be configured to measure blood pressure, preferably also during optimization of the device. The two tools are typically arranged on different body parts, for instance different fingers, which are comparable. This allows a continuous measurement of the blood pressure related parameter, and a continuous optimization of the servo control settings. The optimization therefore does not negatively influence the measurement of the blood pressure related parameter.

### BRIEF DESCRIPTION OF THE DRAWINGS

The invention will be explained by means of the non-limiting working examples depicted in the following figures. Specifically:
- Figure 1 schematically shows a device for non-invasive blood pressure measurements according to the prior art; and
- Figure 2 schematically shows the results of different gains on a modelled finger;

### DETAILED DESCRIPTION

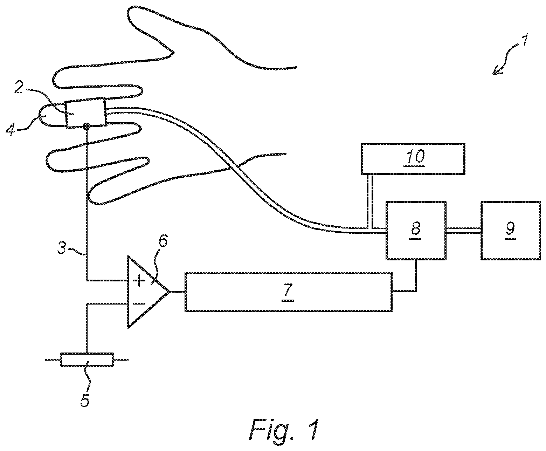

Figure 1 schematically shows a device (1) for non-invasive blood pressure measurements according to the prior art, comprising a pressure cuff (2), which generates a signal (3), the plethysmogram, based on the detected light. This signal (3), representative for the volume of blood in the finger (4) is compared to a set-point (5) by a comparator (6), which comparison is then communicated to a servo controller (7). Based on the information, the servo controller (7) in turn controls a control valve (8). The valve (8) regulates the pressure supplied to the pressure cuff (2) by a pump (9). The pressure supplied to the pressure cuff (2) is measured by a transducer (10). The present invention may be used with a similar device for non-invasive blood pressure measurements, but with an improved method.

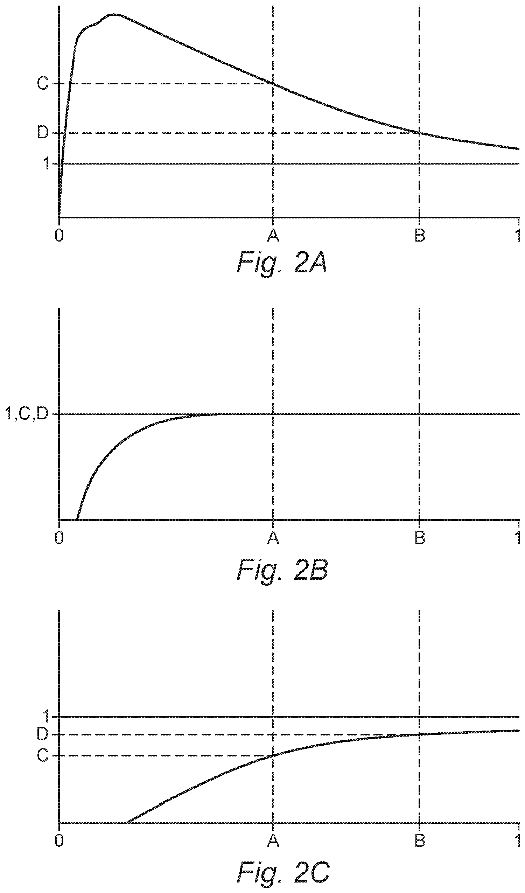

Figure 2A schematically shows a modelled relation between blood pressure related parameter (in this case pulse pressure) and a servo setting (in this case gain) at a first configuration, or first match. The gain of the servo is plotted on the X axis; on the Y axis the pulse pressure is depicted. The line at Y=1 represents the true pulse pressure (as used in the simulation). The curves show the measured pulse pressure (as measured with an instance of a servo with specific settings). The first configuration is a configuration in which the body part is a finger, wherein the finger artery is modelled as a first order Butterworth filter at a -3dB frequency at 1 Hz. Figure 2B shows the same modelled relation with a finger with a -3dBfrequency at 10Hz and figure 2C shows the relation with a finger with a -3dB frequency at 100Hz. In this example, the servo is optimised for a finger with a transfer function similar to a simple first order filter with a -3dB point at 10 Hz. Fig 2B shows that, if the servo is optimised to the finger characteristics changes in gain do not alter the morphology of the blood pressure waveform. Figures 2A and 2C are examples of a mismatch between servo settings and finger characteristics, and changes in gain influence the measured pulse pressure.

On the X-axis of figure 2 a normalized gain is shown, in which the value 1, on the far right, indicates a gain at which the system becomes unstable. On the Y-axis a pulse pressure is shown in arbitrary units. On the X-axis two values (A, B) of normalized gain are indicated, and the corresponding values of the pulse pressure, on the Y-axis are indicated by two other values (C, D) respectively. As can be seen from the figures 2A-2C, the change in gain has significant influence on the Y-axis values in figure 2A (a 1 Hz setting) and figure 2C (a 100Hz setting) and the smallest influence on these values in figure 2B (a 10Hz setting).

It will be apparent that the invention is not limited to the exemplary embodiments shown and described here, but it is defined by the scope of appended claims 1 to 13.

## Claims

1. A method for determining whether a match between the configuration of a servo controller (7) of a non-invasive blood pressure measurement device (1) and characteristics of a body part being measured is optimal or sub-optimal, comprising the steps of:
a. measuring a blood pressure related parameter with a non-invasive blood pressure measurement device (1), which device (1) comprises at least one servo controller (7) in a first configuration and an inflatable pressure cuff (2), wherein inflation of the pressure cuff (2) is controlled by the servo controller (7);
b. changing the settings, in particular the gain, of the servo controller (7)
c. monitoring the change of the blood pressure related parameter under the influence of the changing settings of the servo controller (7); and
d. determining whether a match between the servo controller (7) configuration and characteristics of the body part being measured is optimal or sub-optimal, based on the monitoring of the change of the blood pressure related parameter.

2. Method according to claim 1, wherein i) a match is determined to be optimal when changing the settings, in particular the gain, does not result in a change in the blood pressure related parameter; and wherein ii) a match is determined to be sub-optimal when changing the settings, in particular the gain, does result in a change in the blood pressure related parameter

3. Method according to claim 1 or claim 2, further including the step of:
e. adjusting the configuration of the servo controller (7) in relation to a determination at step d) that the match is sub-optimal.

4. Method according to one of the preceding claims, wherein the blood pressure related parameter is chosen from the group of: pulse pressure, blood pressure or derivatives thereof.

5. Method according to any of the preceding claims, wherein during step b) the settings are changed for at least one heartbeat.

6. Method according to any of the preceding claims, wherein during step b), the settings are changed to a value above the original setting as well as to a value below the original setting.

7. Method according to any of the preceding claims, wherein step d) comprises the steps of calculating the optimal configuration of the servo controller (7) based on the change in settings and resulting change of the blood pressure related parameter and comparing the optimal configuration with the current configuration of the servo controller (7).

8. Method according to any of the preceding claims, including the step of:
f. calculating the optimal configuration of the servo controller (7) based on the change in settings and resulting change of the blood pressure related parameter; and
g. adjusting the configuration of the servo control to the calculated optimal configuration.

9. Method according to any of the preceding claims, wherein steps b) and c) are repeated until the change in settings, in particular the gain, does not result in a change in the blood pressure related parameter, and wherein in step d) an optimal match is established.

10. Method according to any of the preceding claims, wherein the steps are repeated during measurement with the non-invasive blood pressure measurement device (1), in particular periodically.

11. Method according to any of the preceding claims, wherein step b) comprises the steps of normalizing the settings, in particular the gain, of the servo controller (7).

12. Non-invasive blood pressure measurement device (1) comprising a processor configured to execute a method according to any of the preceding claims, and further comprising:
a. at least one servo controller (7);
b. an inflatable pressure cuff (2), wherein inflation of the pressure cuff (2) is controlled by the servo controller (7); and
c. at least one blood pressure measurement tool.

13. Non-invasive blood pressure measurement device according to claim 12, comprising at least two blood pressure measurement tools, wherein a first blood pressure measurement tool is configured for optimization of a non-invasive blood pressure measurement device (1), and wherein a second blood pressure measurement tool is configured to measure blood pressure, preferably also during optimization of the device (1).

## Patentansprüche

1. Verfahren zur Bestimmung, ob eine Übereinstimmung zwischen der Konfiguration einer Servosteuerung (7) einer nichtinvasiven Blutdruckmessvorrichtung (1) und Charakteristika eines Körperteils, welches gemessen wird, optimal oder suboptimal ist, umfassend die Schritte:
a. Messen eines mit Blutdruck zusammenhängenden Parameters mit einer nichtinvasiven Blutdruckmessvorrichtung (1), wobei die Vorrichtung (1) mindestens eine Servosteuerung (7) in einer ersten Konfiguration und eine aufblasbare Druckmanschette (2) umfasst, wobei Aufblasen der Druckmanschette (2) durch die Servosteuerung (7) gesteuert wird;
b. Änderung der Einstellungen, insbesondere des Verstärkungsfaktors, der Servosteuerung (7),
c. Überwachen der Änderung des mit dem Blutdruck zusammenhängenden Parameters unter dem Einfluss der sich ändernden Einstellungen der Servosteuerung (7); und
d. Bestimmen, ob eine Übereinstimmung zwischen der Konfiguration der Servosteuerung (7) und Charakteristika des Körperteils, welches gemessen wird, optimal oder suboptimal ist, bezogen auf die Überwachung der Änderung des mit dem Blutdruck zusammenhängenden Parameters.

2. Verfahren nach Anspruch 1, wobei i) bestimmt wird, dass eine Übereinstimmung optimal ist, wenn Änderung der Einstellungen, insbesondere des Verstärkungsfaktors, nicht zu einer Änderung des mit dem Blutdruck zusammenhängenden Parameters führt; und wobei (ii) bestimmt wird, dass eine Übereinstimmung suboptimal ist, wenn Ändern der Einstellungen, insbesondere des Verstärkungsfaktors, zu einer Änderung des mit dem Blutdruck zusammenhängenden Parameters führt.

3. Verfahren nach Anspruch 1 oder Anspruch 2, des Weiteren umfassend den Schritt:
e. Anpassen der Konfiguration der Servosteuerung (7) im Zusammenhang mit einer Bestimmung in Schritt d), dass die Übereinstimmung suboptimal ist.

4. Verfahren nach einem der vorhergehenden Ansprüche, wobei der mit dem Blutdruck zusammenhängende Parameter ausgewählt ist aus der Gruppe von: Pulsdruck, Blutdruck oder Ableitungen davon.

5. Verfahren nach einem der vorhergehenden Ansprüche, wobei während Schritt b) die Einstellungen für mindestens einen Herzschlag geändert werden.

6. Verfahren nach einem der vorhergehenden Ansprüche, wobei während Schritt b) die Einstellungen auf einen Wert oberhalb der ursprünglichen Einstellung sowie auf einen Wert unterhalb der ursprünglichen Einstellung geändert werden.

7. Verfahren nach einem der vorhergehenden Ansprüche, wobei Schritt d) die Schritte des Berechnens der optimalen Konfiguration der Servosteuerung (7) basierend auf der Änderung in den Einstellungen und der resultierenden Änderung des mit dem Blutdruck zusammenhängenden Parameters sowie Vergleichen der optimalen Konfiguration mit der aktuellen Konfiguration der Servosteuerung (7) umfasst.

8. Verfahren nach einem der vorhergehenden Ansprüche, das den Schritt umfasst:
f. Berechnen der optimalen Konfiguration der Servosteuerung (7) basierend auf der Änderung der Einstellungen und resultierenden Änderung des mit dem Blutdruck zusammenhängenden Parameters; und
g. Anpassen der Konfiguration der Servosteuerung an die berechnete optimale Konfiguration.

9. Verfahren nach einem der vorhergehenden Ansprüche, wobei Schritte b) und c) wiederholt werden, bis die Änderung der Einstellungen, insbesondere des Verstärkungsfaktors, nicht zu einer Änderung des mit dem Blutdruck zusammenhängenden Parameters führt, und wobei in Schritt d) eine optimale Übereinstimmung etabliert wird.

10. Verfahren nach einem der vorhergehenden Ansprüche, wobei die Schritte während der Messung mit der nichtinvasiven Blutdruckmessvorrichtung (1) wiederholt werden, insbesondere periodisch.

11. Verfahren nach einem der vorhergehenden Ansprüche, wobei Schritt b) die Schritte des Normalisierens der Einstellungen, insbesondere des Verstärkungsfaktors, der Servosteuerung (7) umfasst.

12. Nichtinvasive Blutdruckmessvorrichtung (1), umfassend einen Prozessor, der ausgestaltet ist, um ein Verfahren nach einem der vorhergehenden Ansprüche auszuführen, und des Weiteren umfassend:
a. mindestens eine Servosteuerung (7);
b. eine aufblasbare Druckmanschette (2), wobei Aufblasen der Druckmanschette (2) durch die Servosteuerung (7) gesteuert wird; und
c. mindestens ein Blutdruckmesswerkzeug.

13. Nichtinvasive Blutdruckmessvorrichtung nach Anspruch 12, umfassend mindestens zwei Blutdruckmesswerkzeuge, wobei ein erstes Blutdruckmesswerkzeug zur Optimierung einer nichtinvasiven Blutdruckmessvorrichtung (1) ausgestaltet ist, und wobei ein zweites Blutdruckmesswerkzeug ausgestaltet ist, um den Blutdruck zu messen, vorzugsweise ebenfalls während der Optimierung der Vorrichtung (1) .

## Revendications

1. Procédé pour déterminer si une correspondance entre la configuration d'une servocommande (7) d'un dispositif de mesure de pression artérielle non invasive (1) et des caractéristiques d'une partie du corps à mesurer est optimale ou sous-optimale, comprenant les étapes de :
a. mesure d'un paramètre lié à la pression artérielle à l'aide d'un dispositif de mesure de pression artérielle non invasive (1), lequel dispositif (1) comprenant au moins une servocommande (7) dans une première configuration et un brassard gonflable (2), le gonflage du brassard (2) étant commandé par la servocommande (7) ;
b. modification des réglages, en particulier du gain, de la servocommande (7) ;
c. surveillance de la modification du paramètre lié à la pression artérielle sous l'influence de la modification des réglages de la servocommande (7) ; et
d. détermination si la correspondance entre la configuration de la servocommande (7) et des caractéristiques de la partie du corps mesurée est optimale ou sous-optimale, sur la base de la surveillance de la variation du paramètre lié à la pression artérielle.

2. Procédé selon la revendication 1, i) une correspondance étant déterminée comme étant optimale lorsque la modification des réglages, en particulier du gain, n'entraîne pas de modification du paramètre lié à la pression artérielle ; et ii) une correspondance étant déterminée comme étant sous-optimale lorsque la modification des réglages, en particulier du gain, entraîne une modification du paramètre lié à la pression artérielle.

3. Procédé selon la revendication 1 ou la revendication 2, comprenant en outre l'étape de :
e. réglage de la configuration de la servocommande (7) en fonction de la détermination à l'étape d) que la correspondance est sous-optimale.

4. Procédé selon l'une des revendications précédentes, le paramètre lié à la pression artérielle étant choisi dans le groupe de : la pression du pouls, la pression artérielle ou leurs dérivés.

5. Procédé selon l'une quelconque des revendications précédentes, au cours de l'étape b), les paramètres étant modifiés pour au moins un battement de cœur.

6. Procédé selon l'une quelconque des revendications précédentes, au cours de l'étape b), les réglages étant modifiés à une valeur supérieure au réglage initial ainsi qu'à une valeur inférieure au réglage initial.

7. Procédé selon l'une quelconque des revendications précédentes, l'étape d) comprenant les étapes de calcul de la configuration optimale de la servocommande (7) sur la base de la modification des réglages et de la modification résultante du paramètre lié à la pression artérielle et de la comparaison de la configuration optimale avec la configuration actuelle de la servocommande (7).

8. Procédé selon l'une quelconque des revendications précédentes, comprenant l'étape de :
f. calcul de la configuration optimale de la servocommande (7) sur la base de la modification des réglages et de la modification du paramètre lié à la pression artérielle qui en résulte ; et
g. réglage de la configuration de la servocommande à la configuration optimale calculée.

9. Procédé selon l'une quelconque des revendications précédentes, les étapes b) et c) étant répétées jusqu'à ce que la modification des réglages, en particulier du gain, n'entraîne pas de modification du paramètre lié à la pression artérielle, et, à l'étape d), une correspondance optimale étant établie.

10. Procédé selon l'une quelconque des revendications précédentes, les étapes étant répétées pendant la mesure avec le dispositif de mesure de pression artérielle non invasive (1), en particulier périodiquement.

11. Procédé selon l'une quelconque des revendications précédentes, l'étape b) comprenant les étapes de normalisation des réglages, notamment du gain, de la servocommande (7).

12. Dispositif de mesure de pression artérielle non invasive (1) comprenant un processeur configuré pour exécuter un procédé selon l'une quelconque des revendications précédentes, et comprenant en outre :
a. au moins une servocommande (7) ;
b. un brassard gonflable (2), le gonflage du brassard (2) étant commandé par la servocommande (7) ; et
c. au moins un outil de mesure de la pression artérielle.

13. Dispositif de mesure de pression artérielle non invasive selon la revendication 12, comprenant au moins deux outils de mesure de la pression artérielle, un premier outil de mesure de la pression artérielle étant configuré pour l'optimisation d'un dispositif de mesure de pression artérielle non invasive (1), et un second outil de mesure de la pression artérielle étant configuré pour mesurer la pression artérielle, de préférence également pendant l'optimisation du dispositif (1).
